# EUROPEAN PATENT SPECIFICATION

(11) **EP 0 438 217 B1**
(45) Date of publication and mention of the grant of the patent: **11.01.1995**
(21) Application number: 91300103.8
(22) Date of filing: 08.01.1991
(51) Int. Cl.: A61M 16/18

(54) **Improvements in anaesthetic vaporisers**
Verdampfer für anästhetische Zwecke
Vaporiseurs anesthésiques

(30) Priority: 09.01.1990 GB 9000420
(43) Date of publication of application: 24.07.1991
(73) Proprietor: The BOC Group plc, Windlesham Surrey GU20 6HJ (GB)
(72) Inventor: Montgomery, Frederick John, Bradford West Yorkshire BD15 OHD (GB)
(74) Representative: Gough, Peter

(56) References cited:
- EP-A- 0 339 828
- FR-A- 1 589 569
- GB-A- 2 097 272
- US-A- 4 881 541

## Description

The present invention relates to anaesthetic vaporisers.

UK Patent No. 1 224 478, describes an anaesthetic vaporiser of the by-pass type in which a carrier gas such as oxygen, air or nitrous oxide is initially divided on entry to the vaporiser between a first stream which is directed towards the sump or vaporising chamber of the vaporiser to entrain vapour from a volatile liquid anaesthetic contained therein; and a second by-pass stream, the first and second streams subsequently recombining prior to leaving the vaporiser for delivery to a patient.

This known vaporiser has been used successfully over a number of years for delivering anaesthetic agents such as halothane, trichloroethlene and ether derivatives including enflurane, fluoroxene, methoxyflurane and isoflurane. All the aforementioned anaesthetic agents have a boiling point at atmospheric pressure well above 40°C.

However, a new anaesthetic agent has been developed namely 2-(difluoromethoxy)-1,1,1,2-tetrafluoroethane which has a boiling point at atmospheric pressure of between 20° and 25°C. This physical characteristic of 2-(difluoromethoxy)-1,1,1,2-tetrafluoroethane renders existing anaesthetic vaporisers unsuitable for delivering said agent to a patient.

Conventional vaporisers of the by-pass type are unsuitable for this new anaesthetic agent in that its boiling point is approximately in the middle of a conventional vaporisers operating ambient temperature range of between 15°C and 35°C. When the ambient temperature and hence the vaporiser temperature is above 25°C heat is transferred to the anaesthetic agent and causes an amount of vapour to boil off such that heat lost by the latent heat of vaporisation is equal to the heat transferred to it.

In conventional vaporisers the heat transfer to the anaesthetic agent in the vaporising chamber can be high because of the materials used and this can cause a high volume of anaesthetic agent to boil off. This anaesthetic vapour can leave the vaporiser via the control valve and/or the by-pass valve of the vaporiser.

French Patent No. 1589569 describes a further anaesthetic vaporiser of the by-pass type which includes an inlet for delivery of a carrier gas into the vaporiser and an outlet from the vaporiser for the delivery of carrier gas and anaesthetic agent to a patient. A by-pass passage extends between the inlet and the outlet and located therein is a flow restrictor in the form of an adjustable throttle. Further, a passageway extends between the inlet and the outlet, the passageway having in it a vaporising chamber and a control valve, again in the form of an adjustable throttle. The throttle in the by-pass passage is adjusted so that it closes at a predetermined temperature to cause the entire carrier gas stream to flow through the vaporising chamber.

US Patent No. 4881541 discloses an anaesthetic vaporiser in which components of the vaporiser are maintained at a constant controlled temperature which is greater than ambient temperature by means of a heat source.

It is an aim of the present invention to provide an anaesthetic vaporiser which is capable of delivering a predetermined concentration of an anaesthetic vaporiser having a boiling point at normal atmospheric pressure of less than 30°C to a patient.

According to the present invention, an anaesthetic vaporiser of the by-pass type comprises:
(a) an inlet for delivery of carrier gas into the vaporiser;
(b) an outlet for delivery of carrier gas and anaesthetic agent from the vaporiser to a patient;
(c) a by-pass passage extending between the inlet and the outlet, and a flow restrictor located in the by-pass passage;
(d) a passageway extending between the inlet and the outlet, the passageway having in it (i) a vaporising chamber and (ii) a control valve;
characterised in that the vaporising chamber is thermally insulated and in that a temperature controlling device is located within the insulated vaporising chamber, which device includes an element for cooling anaesthetic agent located within the chamber when the vaporiser is in use, and an element for heating the said anaesthetic agent.

An embodiment of the invention will now be described, by way of example, reference being made to the Figures of the accompanying diagrammatic drawings in which:-
Figure 1 is a diagrammatic sketch of an anaesthetic vaporiser; and
Figure 2 is a side view partly in cross-section of the anaesthetic vaporiser of Figure 1.

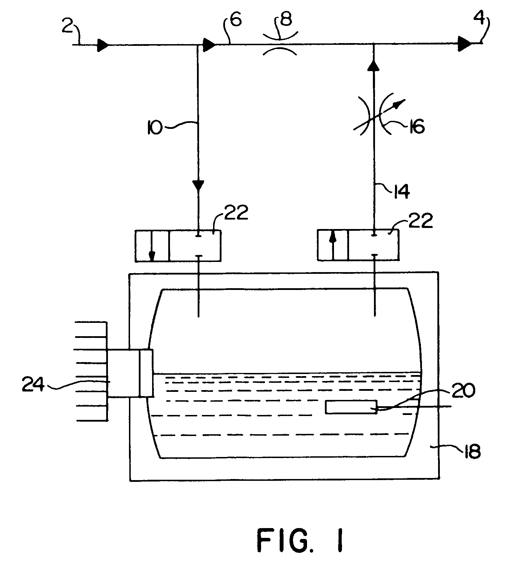

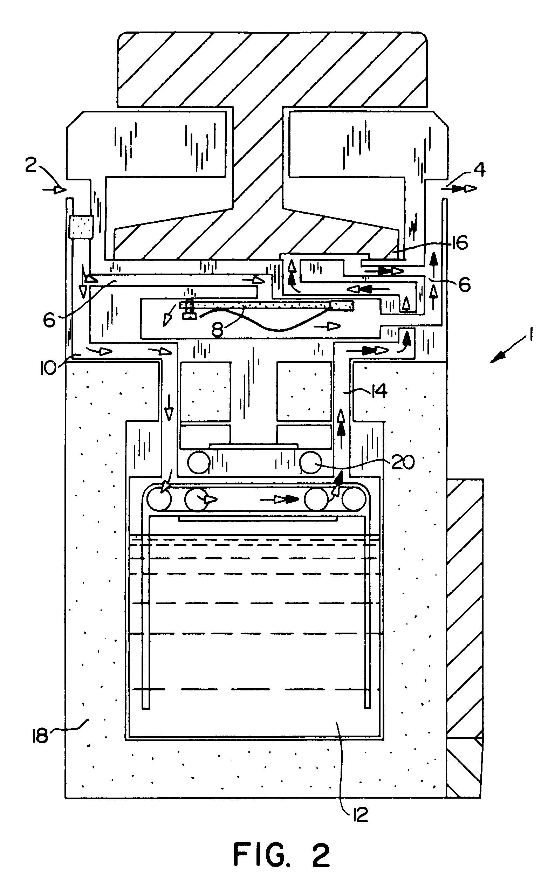

As shown in Figures 1 and 2 an anaesthetic vaporiser 1 of the by-pass type includes a carrier gas inlet 2 and a gas and vapour outlet 4.

A by-pass passage 6 interconnects said inlet 2 and outlet 4 and located in said passage 6 is a laminar flow restrictor 8. The flow restrictor 8 exhibits laminar flow characteristics over its full operating range.

A passage 10 extends from passage 6 to the interior of a vaporising chamber 12 containing and anaesthetic agent having a boiling point at normal atmospheric pressure of 30°C or less e.g., 2-(difluoromethoxy)-1,1,1,2-tetrafluoroethane. A passage 14 extends from the interior of the vaporising chamber 12 back to the passage 6 and located in said passage 14 is a laminar control valve 16. The laminar control valve 16 exhibits laminar flow characteristics over its full operating range.

The passages 10, 14; the vaporising chamber and the control valve 16 define a passageway between the inlet 2 and the outlet 4.

The vaporising chamber 12 is substantially surrounded by thermal insulation material 18 which can be in the order of 20 millimetres thick to prevent or inhibit uncontrolled boiling.

Located within the vaporiser 1 is a temperature controlled heater 20 and a Peltier device 24 which when supplied with a suitable electric current can cool or heat the anaesthetic agent contained within the vaporising chamber 12.

Arranged in each passage 10, 14 is an isolator 22.

In use, the control valve 16 is set in a manner known per se to split the flow of carrier gas entering inlet 2 between a first stream which is directed along passage 10 to the interior of the vaporising chamber 12 and a second by-pass stream which flows directly along passage 6 to the outlet 4.

The first stream enters the vaporising chamber 12 and entrains vapour of the anaesthetic agent contained therein and the carrier gas and vapour mixture leaves the vaporising chamber 12 via passage 14 to rejoin the second stream in the passage 6 prior to leaving the anaesthetic vaporiser 1 at the outlet 4.

The above described vaporiser is of the by-pass type and has a thermally insulated vaporising chamber 12. The insulating material 18 limits the heat transfer into the vaporising chamber 12 when the outside ambient temperature is greater than the vaporiser's designed operating temperature which is below the boiling point of the anaesthetic agent.

When in use, the anaesthetic agent is vaporised and the heat removed from the vaporising chamber is equivalent to the latent heat of vaporisation.

If the amount lost via latent heat of vaporisation is greater than the heat transfer coming from the outside then the temperature in the vaporising chamber 12 will fall.

The temperature controlled heater 20 and/or the Peltier device 24 prevents the temperature in the vaporising chamber 12 falling below a set value and provides the heat required to evaporate the anaesthetic agent needed for the carrier gas flow and high concentration settings of the vaporiser 1.

The amount of insulating material 18 is specified to ensure that the heat lost through latent heat of vaporisation is always greater than that gained from heat transfer through the vaporising chamber walls over the required range of carrier gas flow rates and concentration settings.

When the anaesthetic agent is 2-(difluoromethoxy)-1,1,1,2-tetrafluoroethane, if the minimum flow and concentration are specified as one litre per minute and 1% concentration by volume of agent respectively then the latent heat loss from vaporisation under these conditions would be approximately 0.17 watts. If the operating temperature of the vaporising chamber 12 is designed to be 22°C and the maximum ambient temperature for operating the vaporiser 1 is 30°C then a typical thickness of expanded polystyrene insulating material 18 would need to be in the order of 20mm.

If the vaporiser control valve 16 is left in an on position and there is no carrier gas flow and the ambient temperature is above the boiling point of the anaesthetic agent then vapour equivalent to the heat transferred to the vaporising chamber 12 will boil off and could slowly fill the anaesthesia machine's gas circuits with anaesthetic vapour. This could give a dangerous concentration to a patient if connected to the circuit and the carrier gas were to be turned on.

To prevent this situation the isolators 22 can be actuated to isolate the vaporising chamber from the rest of the vaporiser when there is no carrier gas flow. When the vaporiser 1 is in the off position, the vaporising chamber 12 can be isolated by the control valve 16. However, heat transfer to the vaporising chamber 12 can still occur if there is a high ambient temperature.

There are a number of ways to accommodate this situation.

First, when in the off position the vaporising chamber 12 could be vented to atmosphere in which case the anaesthetic vapour equivalent to the heat transfer would boil off to atmosphere and would be wasted but the temperature and pressure in the vaporising chamber 12 would remain at the boiling point of the anaesthetic agent about 22°C and ambient pressure respectively. When turned on the vaporiser 1 would operate normally straight away with little deviation from the set concentration.

Secondly, the vaporising chamber 12 could be isolated when the control vale 16 is in the off position. In this case the vaporising chamber 12 will slowly rise in temperature to that of the ambient temperature and the pressure in the vaporising chamber 12 will rise above ambient pressure in line with the anaesthetic agents saturated vapour pressure characteristics. When turned on the vaporiser 1 would boil off vapour until the temperature and pressure in the vaporising chamber 12 came down to normal level.

This could result in an initially high concentration of anaesthetic being delivered to the patient. However, it could be arranged so that this initial bolus of vapour was vented off to atmosphere before connecting the vaporising chamber 12 with the rest of the vaporiser gas passage.

Thirdly, and most advantageously rather than venting off the anaesthetic when the vaporiser 1 is turned on the Peltier device 24 can be used to cool the vaporising chamber 12 down to its operating temperature before the vaporising chamber is opened and the vaporiser is operational.

The Peltier device 24 can keep the vaporising chamber 12 cool which will minimise the requirement for thermal insulation 18.

## Claims

1. An anaesthetic vaporiser (1) of the by-pass type comprising:
(a) an inlet (2) for delivery of carrier gas into the vaporiser;
(b) an outlet (4) for delivery of carrier gas and anaesthetic agent from the vaporiser to a patient;
(c) a by-pass passage (6) extending between the inlet (2) and the outlet (4), and a flow restrictor (8) located in the by-pass passage;
(d) a passageway (10) extending between the inlet (2) and the outlet (4), the passageway having in it (i) a vaporising chamber (12) and (ii) a control valve (16);
characterised in that the vaporising chamber (12) is thermally insulated and in that a temperature controlling device (20, 24) is located within the insulated vaporising chamber (12), which device (24) includes an element for cooling anaesthetic agent located within the chamber (12) when the vaporiser is in use, and an element for heating the said anaesthetic agent.

2. A vaporiser as claimed in Claim 1 , characterised in that the elements for heating the anaesthetic agent and cooling the anaesthetic agent respectively are provided by a single element.

3. A vaporiser as claimed in Claim 1 or 2, characterised in that the temperature controlling device includes a temperature controlling Peltier device (24).

4. A vaporiser as claimed in Claim 3, characterised in that the temperature controlling device includes a supplementary heater (20) for the anaesthetic agent, in addition to the said element for heating.

5. A vaporiser as claimed in any one of Claims 1 to 4, which includes isolators (22) located in the said passageway (10) by which flow of carrier gas to, and flow of carrier gas and anaesthetic agent from, the vaporising chamber (12) can be prevented.

6. A vaporiser as claimed in any one of Claims 1 to 5, in which the flow restrictor (8) and the control valve (16) are substantially laminar flow devices over their full operating ranges.

## Patentansprüche

1. Ein Narkosemittelverdampfer (1) des Umgehungstypus mit:
(a) einem Einlaß (2) zum Liefern von Trägergas in den Verdampfer;
(b) einem Auslaß (4) zum Liefern von Trägergas und Narkosemittelagens aus dem Verdampfer an einen Patienten;
(c) einem Umgehungsdurchtritt (6), der sich zwischen dem Einlaß (2) und dem Auslaß (4) erstreckt und einem Flußbegrenzer (8), der in dem Umgehungsdurchtritt angeordnet ist;
(d) einem Durchtrittsweg (10), der sich zwischen dem Einlaß (2) und dem Auslaß (4) erstreckt, wobei der Durchtrittsweg darin (i) eine Verdampfungskammer (12) und (ii) ein Steuerventil (16) aufweist;
dadurch gekennzeichnet, daß die Verdampfungskammer (12) thermisch isoliert ist und dadurch, daß eine temperaturregelnde Vorrichtung (20, 24) innerhalb der isolierten Verdampfungskammer (12) angeordnet ist, welche Vorrichtung (24) ein Element zum Kühlen von Narkosemittelagens, das innerhalb der Kammer (12) angeordnet ist, wenn der Verdampfer in Benutzung ist, und ein Element zum Wärmen des Narkosemittelagens umfaßt.

2. Ein Verdampfer nach Anspruch 1, dadurch gekennzeichnet, daß die Elemente zum Erwärmen des Narkosemittelagens bzw. zum Kühlen des Narkosemittelagens durch ein einzelnes Element vorgesehen werden.

3. Ein Verdampfer nach Anspruch 1 oder 2, dadurch gekennzeichnet, daß die temperaturregelnde Vorrichtung eine temperaturregelnde Peltier-Vorrichtung (24) umfaßt.

4. Ein Verdampfer nach Anspruch 3, dadurch gekennzeichnet, daß das temperaturregelnde Gerät einen ergänzenden Heizer (20) für das Narkosemittelagens zusätzlich zu dem Element zum Erwärmen umfaßt.

5. Ein Verdampfer nach einem der Ansprüche 1 bis 4, welcher Isolatoren (22), die in dem Durchtrittsweg (10) angeordnet sind, umfaßt, durch welche Fluß des Trägergases zu und Fluß von Trägergas und Narkosemittelagens aus der Verdampfungskammer (12) verhindert werden kann.

6. Ein Verdampfer nach einem der Ansprüche 1 bis 5, in welchem der Flußbegrenzer (8) und das Steuerventil (16) im wesentlichen Laminarflußgeräte über ihre vollen Betriebsbereiche sind.

## Revendications

1. Vaporisateur (1) d'anesthésique du type à dérivation comprenant :
(a) une entrée (2) pour délivrer un gaz porteur dans le vaporisateur ;
(b) une sortie (4) pour fournir depuis le vaporisateur le gaz porteur et un agent anesthésique à un patient ;
(c) un passage de dérivation (6) s'étendant entre l'entrée (2) et la sortie (4), et un limiteur d'écoulement (8) situé dans le passage de dérivation;
(d) un passage (10) s'étendant entre l'entrée (2) et la sortie (4), le passage possédant intérieurement (i) une chambre de vaporisation (12) et (ii) une vanne de contrôle (16);
***caractérisé en ce que*** la chambre de vaporisation (12) est isolée thermiquement et ***en ce qu***'un dispositif de contrôle de la température (20, 24) est situé à l'intérieur de la chambre de vaporisation isolée (12), lequel dispositif (24) inclut un élément pour refroidir l'agent anesthésique situé dans la chambre (12) lorsque le vaporisateur est en utilisation, et un élément pour chauffer ledit agent anesthésique.

2. Vaporisateur selon la Revendication 1, ***caractérisé en ce que*** les éléments pour respectivement chauffer l'agent anesthésique et refroidir l'agent anesthésique sont procurés par un seul élément.

3. Vaporisateur selon la Revendication 1 ou 2, ***caractérisé en ce que*** le dispositif de contrôle de la température inclut un dispositif de Peltier de contrôle de la température (24).

4. Vaporisateur selon la Revendication 3, ***caractérisé en ce que*** le dispositif de contrôle de la température inclut un réchauffeur supplémentaire (20) pour l'agent anesthésique, en plus dudit élément de chauffage.

5. Vaporisateur selon l'une quelconque des Revendications 1 à 4, qui comprend des isolateurs (22) situés dans ledit passage (10) par lesquels l'écoulement de gaz porteur vers, et l'écoulement de gaz porteur et d'agent anesthésique depuis, la chambre de vaporisation (12) peuvent être empêchés.

6. Vaporisateur selon l'une quelconque des Revendications 1 à 5, dans lequel le limiteur d'écoulement (8) et la vanne de contrôle (16) sont des dispositifs d'écoulement substantiellement laminaire sur l'intégralité de leur domaine de fonctionnement.
